(19) Europäisches Patentamt
European Patent Office
Office européen des brevets

(11) **EP 2 332 928 A2**

(12) **EUROPEAN PATENT APPLICATION**

(43) Date of publication:
**15.06.2011 Bulletin 2011/24**

(51) Int Cl.:
*C07D 307/50* [(2006.01)]   *C13K 1/02* [(2006.01)]

(21) Application number: **10190871.3**

(22) Date of filing: **11.11.2010**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **01.12.2009 JP 2009273471**

(71) Applicant: **Honda Motor Co., Ltd.**
**Tokyo 107-8556 (JP)**

(72) Inventor: **Miyashita, Kohichi**
**Saitama 351-0193 (JP)**

(74) Representative: **Böhm, Brigitte et al**
**Weickmann & Weickmann**
**Patentanwälte**
**Postfach 86 08 20**
**81635 München (DE)**

(54) **Method for lysing cellulose**

(57)    A method for obtaining a saccharide by lysing cellulose which is substance that is not readily lysed. Cellulose is mixed in acidic electrolyzed water, and the resulting mixture is stirred at a maximum temperature of 210°C and at saturation vapor pressure of 1.9 MPa to obtain a saccharide.

## FIG.1

EP 2 332 928 A2

**Description**

The present invention relates to a method for lysing cellulose to manufacture saccharides or hydroxymethylfurfural (HMF).

Obtaining saccharides or other substances from the cellulose contained in trees would be beneficial for human food resources. However, cellulose is a substance that is not readily lysed. Accordingly, a variety of lysing methods have been proposed in the past; e.g., as disclosed in Japanese Patent Application Laid-Open Publication No. 2001-095594 (JP 2001-095594 A).

In the method for lysing cellulose disclosed in JP 2001-095594 A, cellulose is solubized using supercritical water or subcritical water, a cellulase preparation is then introduced, whereupon hydrolysis is performed to yield glucose and/or a cello oligosaccharide. The temperature of the supercritical water or subcritical water is disclosed as 320 to 500°C, and the pressure as 20 to 50 MPa.

Cellulose must be processed at very high temperatures and pressure; i.e., at or above 320°C and 20 MPa (about 204 kgf/cm$^2$) because it is a substance that is not readily lysed. However, a substantial amount of energy is required to keep the temperature high, and pressure-resistant equipment is required to keep the pressure high. Accordingly, the method disclosed in JP 2001-095594 A entails an increase in the equipment procurement cost as well as an increase in running costs.

The current need to conserve resources has created a demand for a cellulose-lysing method that allows equipment procurement costs and running costs to be reduced.

It is an object of the present invention to provide a cellulose-lysing method that allows equipment procurement costs and running costs to be reduced.

According to a first aspect of the present invention, there is provided a method for lysing cellulose, comprising the steps of mixing cellulose in acidic electrolyzed water; and stirring the resulting mixture at a maximum temperature of 210°C and at saturation vapor pressure to obtain a saccharide.

In the present invention, energy can be conserved because the temperature can be dramatically reduced (e.g., the conventional temperature of 320°C can be reduced to 210°C in the present invention), and the cost of procuring processing equipment can be dramatically reduced because the pressure can be dramatically reduced (e.g., the conventional pressure of 20 MPa can be reduced to 1.9 MPa in the present invention).

According to another aspect of the present invention, there is provided a method for lysing cellulose, comprising the steps of mixing cellulose in acidic electrolyzed water; and stirring the resulting mixture at a maximum temperature of 200°C and a maximum pressure of 2.0 MPa to obtain a saccharide.

In the present invention, energy can be conserved because the temperature can be dramatically reduced (e.g., the conventional temperature of 320°C can be reduced to 200°C in the present invention), and the cost of procuring processing equipment can be dramatically reduced because the pressure can be dramatically reduced (e.g., the conventional pressure of 20 MPa can be reduced to 2.0 MPa in the present invention).

According to a further aspect of the present invention, there is provided a method for lysing cellulose, comprising the steps of mixing cellulose in electrolyzed water containing a hydroxyl radical; and stirring the resulting mixture at a maximum temperature of 230°C and at saturation vapor pressure to obtain hydroxymethylfurfural.

In the present invention, energy can be conserved because the temperature can be dramatically reduced (e.g., the conventional temperature of 320°C can be reduced to 230°C in the present invention), and the cost of procuring processing equipment can be dramatically reduced because the pressure can be dramatically reduced (e.g., the conventional pressure of 20 MPa can be reduced to 2.8 MPa in the present invention).

Hydroxymethylfurfural (HMF) is a pharmaceutical ingredient that holds promise in terms of its effect on lowering blood pressure, ability to relieve hypertension, and remedial effect on diabetes and the like. HMF has been costly in the past, but the present invention enables the cost of manufacturing HMF to be reduced, provides beneficial pharmaceuticals at low cost, and aims to contribute to the societal good.

Certain preferred embodiments of the present invention will be described in detail below, by way of example only, with reference to the accompanying drawings, in which:

FIG. 1 is a schematic view illustrating an apparatus for producing acidic electrolyzed water used in the present invention;
FIG. 2 is a view illustrating the basic arrangement of an apparatus for lysing cellulose;
FIG. 3 is a graph showing a correlation between pressures and glucose produced;
FIG. 4 is a schematic view of an apparatus for producing electrolyzed water containing hydroxyl radicals, as used in the present invention; and
FIG. 5 is a chromatograph view showing the intensity of an HMF signal.

(First Embodiment)

First, there will be provided a description of the principle employed in Example 1 for manufacturing acidic electrolyzed water, which is an important substance.

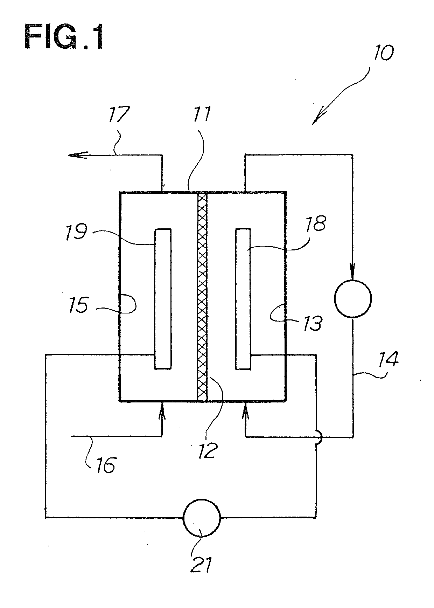

As shown in FIG. 1, an apparatus 10 for producing acidic electrolyzed water comprises an electrolyzer 11, an anion exchange membrane 12 for dividing the electrolyzer 11 into right and left compartments, an aqueous solution circulating mechanism 14 for circulating an aqueous solution through a right chamber 13, a water-supply tube 16 for supplying tap water to a left chamber 15, an electrolyzed-water-removal tube 17 for removing acidic electrolyzed water from the left chamber 15, a cathode electrode 18 accommodated in the right chamber 13, an anode electrode 19 accommodated in the left chamber 15, and a power source 21 for applying a predetermined voltage to the electrodes 18, 19.

The right chamber 13 was filled with an aqueous solution of sodium chloride (NaCl), and the solution was circulated by the aqueous solution circulating mechanism 14.

The left chamber 15 was filled with tap water ($H_2O$ + Cl-). A predetermined voltage was then applied to the electrodes 18, 19 by the power source 21.

The sodium chloride (NaCl) was then broken down in the right chamber 13, and sodium ions ($Na^+$) and chlorine ions (Cl-) were produced.

Only anions are allowed to pass through the anion exchange membrane 12; therefore, the chlorine ions (Cl-) migrated to the left chamber 15.

The chlorine ions (Cl-) that migrated from the right chamber 13 were added to the chlorine ions (Cl-) contained in the tap water in the left chamber 15, the concentration of chlorine ions (Cl-) increased, and the following reaction occurred in the presence of the water ($H_2O$) contained in the tap water.

$$2Cl^- \rightarrow Cl_2 + 2e^-$$

$$Cl_2 + 2H_2O \rightarrow 2HClO + 2H^-$$

$$H_2O \rightarrow \frac{1}{2}O_2 + 2H^+ + 2e^-$$

Specifically, chlorine ($Cl_2$) was produced from the chlorine ions (Cl-). The chlorine ($Cl_2$) reacted with the water to produce hypochlorous acid (HClO). The water underwent electrolysis to produce oxygen ($O_2$) and hydrogen ions (H-).

As a result, the acidic electrolyzed water containing hydrogen ions (H-) and hypochlorous acid (HClO) could be removed via the electrolyzed-water-removal tube 17. The acidic electrolyzed water is sodium-free acidic electrolyzed water; i.e., contains no sodium.

The basic structure of a cellulose-lysing apparatus will now be described in the following.

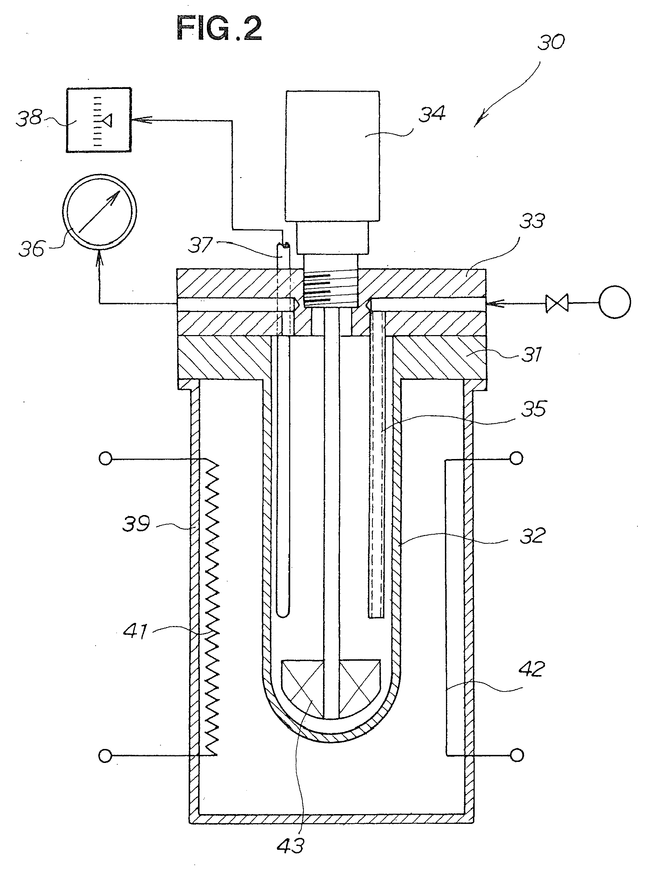

As shown in FIG. 2, a cellulose-lysing apparatus 30 comprises a cylindrical pressure vessel 32, an upper part thereof being open and having a flange 31, and a lower part having a hemispherical shell-shaped bottom; a cover 33 for covering the opening on the upper part of the pressure vessel 32; a stirring motor 34, a liquid supply tube 35, a pressure gauge 36, and a thermocouple protection tube 37, each of which being provided to the cover 33; a thermometer 38 for converting electrical information from the thermocouple accommodated in the thermocouple protection tube 37 to temperature information; a jacket 39 for enclosing the pressure vessel 32; a heater 41 and a water cooling tube 42, each of which being attached to the jacket 39; and a stirring blade 43 suspended from the stirring motor 34.

Granular cellulose was introduced into the pressure vessel 32, and the cover 33 was closed. A predetermined amount of acidic electrolyzed water was then supplied through the liquid supply tube 35. The resulting mixture was heated by the heater 41, and stirred by the stirring blade 43 while the temperature was monitored by the thermometer 38 and the pressure was monitored by the pressure gauge 36. An inert-gas injection tube or a pressure-release tube was preferably attached in order to regulate the pressure.

Experimental examples carried out using the apparatus will now be described.

Experimental Examples

An experimental example according to the present invention will be described below; however, the present invention is not limited to the experimental example.

• Mixing step

Cellulose: microcrystalline, 2 g
Electrolyzed water: sodium-free acidic electrolyzed water or purified water, 98 g
The purified water was obtained by treating water with a reverse osmosis membrane, and was prepared for comparative experiments.

• Experiment to identify ideal temperature

The pressure was held at saturation vapor pressure while the temperature was varied among 170, 200, 210, 220, and 230°C, whereby the resulting amount of glucose was examined. The results are shown in Table 1 below.

Table 1

| Experiment number | Mixture | | Processing conditions | | | Glucose produced |
|---|---|---|---|---|---|---|
| | Cellulose | Water | Temperature | Pressure | Time | |
| Experiment 1 | 2 g | Purified Water 98 g | 170°C | Saturation vapor pressure (0.8 MPa) | 30 min | 0 Ppm |
| Experiment 2 | 2 g | Purified Water 98 g | 200°C | Saturation vapor pressure (1.6 MPa) | 30 min | Ppm 8 |
| Experiment 3 | 2 g | Acidic electrolyzed water 98 g | 170°C | Saturation vapor pressure (0.8 MPa) | 30 min | 527 Ppm |
| Experiment 4 | 2 g | Acidic electrolyzed water 98 g | 200°C | Saturation vapor pressure (1.6 MPa) | 30 min | 4727 Ppm |
| Experiment 5 | 2 g | Acidic electrolyzed water 98 g | 210°C | Saturation vapor pressure (1.9 MPa) | 30 min | 12,152 Ppm |
| Experiment 6 | 2 g | Acidic electrolyzed water 98 g | 220°C | Saturation vapor pressure (2.3 MPa) | 30 min | 10,712 Ppm |
| Experiment 7 | 2 g | Acidic electrolyzed water 98 g | 230°C | Saturation vapor pressure (2.8 MPa) | 30 min | 274 Ppm |

In Experiment 1, 98 g of purified water was added to 2 g of cellulose as a control, and the mixture was stirred for 30 minutes at 170°C and at saturation vapor pressure (0.8 MPa). Glucose was not produced.
In Experiment 2, 98 g of purified water was added to 2 g of cellulose as a control, and the mixture was stirred for 30 minutes at 200°C and at saturation vapor pressure (1.6 MPa), resulting in 8 ppm of glucose.
In Experiment 3, 98 g of acidic electrolyzed water according to the present invention was added to 2 g of cellulose, and the mixture was stirred for 30 minutes at 170°C and at saturation vapor pressure (0.8 MPa), resulting in 527 ppm of glucose.
In Experiment 4, 98 g of acidic electrolyzed water according to the present invention was added to 2 g of cellulose, and the mixture was stirred for 30 minutes at 200°C and at saturation vapor pressure (1.6 MPa), resulting in 4727 ppm of glucose.
In Experiment 5, 98 g of acidic electrolyzed water according to the present invention was added to 2 g of cellulose, and the mixture was stirred for 30 minutes at 210°C and at saturation vapor pressure (1.9 MPa), resulting in 12,152 ppm of glucose.
In Experiment 6, 98 g of acidic electrolyzed water according to the present invention was added to 2 g of cellulose, and the mixture was stirred for 30 minutes at 220°C and at saturation vapor pressure (2.3 MPa), resulting in 10,712 ppm of glucose.

In Experiment 7, 98 g of acidic electrolyzed water according to the present invention was added to 2 g of cellulose, and the mixture was stirred for 30 minutes at 230°C and at saturation vapor pressure (2.8 MPa), resulting in 274 ppm of glucose.

The following could be determined from the preceding experiments.

A cellulolytic action was substantially indiscernible in the purified water used in Experiments 1 and 2. However, a cellulolytic action was identified in the acidic electrolyzed water used in Experiments 3 through 7.

Among Experiments 3 through 7, Experiment 5 (210°C) was optimal, followed by Experiment 6 (220°C) and then Experiment 4 (200°C).

However, Experiment 4 (200°C) produced better results than Experiment 7 (230°C). Accordingly, it could be confirmed that, based on saturation vapor pressure, a range of 200 to 230°C is preferable, and 210°C is the ideal temperature.

As described in the prior art section, cellulose, a substance not readily lysed, has conventionally been processed at high temperatures (at or above 320°C) and high pressure (at or above 20 MPa).

However, according to the present invention, in which acidic electrolyzed water is used, cellulose can be processed at saturation vapor pressure (1.9 MPa) at a maximum temperature of 210°C.

Energy can be conserved because the temperature can be dramatically reduced (320°C to 210°C), and the cost of procuring processing equipment can be dramatically reduced because the pressure can be dramatically reduced (20 MPa to 1.9 MPa).

An ideal pressure was investigated, as described below, at a fixed temperature and using acidic electrolyzed water.

• Experiment to identify ideal pressure

The temperature was held at 200°C while the pressure was varied among 2.0, 2.5, 3.0, and 5.0 MPa, whereby the amount of glucose produced was examined. The results are shown in Table 2 below.

Table 2

| Experiment number | Mixture | | Processing conditions | | | Glucose produced |
|---|---|---|---|---|---|---|
| | Cellulose | Water | Temperature | Pressure | Time | |
| Experiment 4 | 2 g | Acidic electrolyzed Water 98 g | 200°C | Saturation vapor pressure (1.6 MPa) | 30 min | 4727 ppm |
| Experiment 8 | 2g | Acidic electrolyzed water 98 g | 200°C | 2.0 MPa | 30 min | 9537 ppm |
| Experiment 9 | 2 g | Acidic electrolyzed water 98 g | 200°C | 2.5 MPa | 30 min | 9779 ppm |
| Experiment 10 | 2 g | Acidic electrolyzed water 98 g | 200°C | 3.0 MPa | 30 min | 10,448 ppm |
| Experiment 11 | 2 g | Acidic electrolyzed water 98 g | 200°C | 5.0 MPa | 30 min | 10,313 ppm |

Experiment 4 is a reproduction of Experiment 4 in Table 1.

In Experiment 8, 98 g of acidic electrolyzed water according to the present invention was added to 2 g of cellulose, and the mixture was stirred for 30 minutes at 200°C and 2.0 MPa, resulting in 9537 ppm of glucose.

In Experiment 9, 98 g of acidic electrolyzed water according to the present invention was added to 2 g of cellulose, and the mixture was stirred for 30 minutes at 200°C and 2.5 MPa, resulting in 9779 ppm of glucose.

In Experiment 10, 98 g of acidic electrolyzed water according to the present invention was added to 2 g of cellulose, and the mixture was stirred for 30 minutes at 200°C and 3.0 MPa, resulting in 10,448 ppm of glucose.

In Experiment 11, 98 g of acidic electrolyzed water according to the present invention was added to 2 g of cellulose, and the mixture was stirred for 30 minutes at 200°C and 5.0 MPa, resulting in 10,313 ppm of glucose.

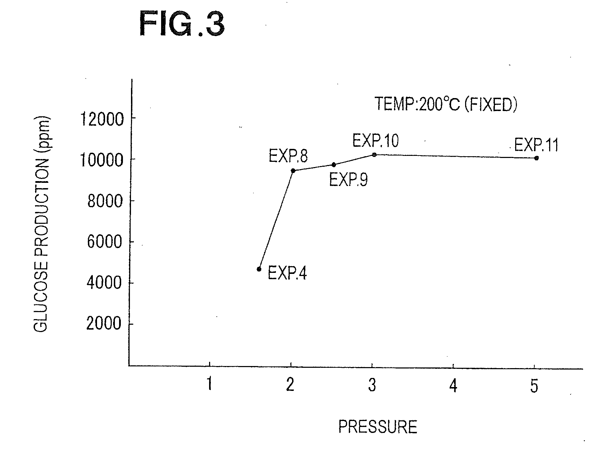

The correlation between the pressure and the glucose produced is shown in FIG. 3.

The amount of glucose produced in Experiment 8 (2.0 MPa) was double that of Experiment 4 (1.6 MPa), as shown in the graph in FIG. 3. An increase in pressure was confirmed to be related to an increase in the amount of glucose produced. However, the increase was not to the extent expected in Experiment 9 (2.5 MPa) and Experiment 10 (3 MPa). In Experiment 11 (5 MPa), the amount of glucose was not noted to increase, and instead tended to decrease.

Accordingly, it could be confirmed that at 200°C the ideal pressure is 2.0 MPa.

As described in the prior art section, cellulose, a substance not readily lysed, has conventionally been processed at high temperatures (at or above 320°C) and high pressure (at or above 20 MPa).

However, according to the present invention, in which acidic electrolyzed water is used, cellulose can be processed at a maximum temperature of 200°C and a maximum pressure of 2.0 MPa.

Energy can be conserved because the temperature can be dramatically reduced (320°C to 200°C), and the cost of procuring processing equipment can be dramatically reduced because the pressure can be dramatically reduced (20 MPa to 2.0 MPa).

(Second Embodiment)

A separate experiment was performed in Example 2 to determine whether or not replacing the acidic electrolyzed water used in Experimental Example 1 with electrolyzed water containing hydroxyl radicals would produce a functional effect.

There will now be provided a description of the principle employed in Example 2 for manufacturing electrolyzed water containing hydroxyl radicals (also referred to as "OH radicals" below), which is an important substance.

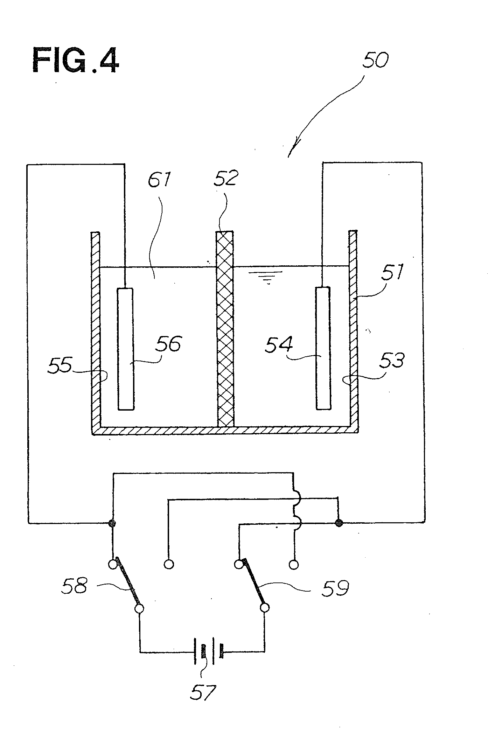

As shown in FIG. 4, an apparatus 50 for producing OH-radical-electrolyzed water comprises an electrolyzer 51, an ion-permeable membrane 52 for dividing the electrolyzer 51 into right and left compartments, an electrode 54 accommodated in a right chamber 53, an electrode 56 accommodated in a left chamber 55, a power source 57 for applying a predetermined voltage to the electrodes 54, 56, and switches 58,59.

Raw water 61 containing sodium chloride or potassium chloride was supplied to the electrolyzer 51. When the switches 58, 59 were flipped to the left as shown in the drawing, the left electrode 56 became an anode electrode, and the right electrode 54 became a cathode electrode.

The reaction shown in the following then occurred in the right chamber 53, which is the cathode side.

$$2H_2O + 2e^- \rightarrow 2OH^- + H_2$$

Specifically, hydroxide ions (OH-) were produced in the right chamber 53.

When the switches 58, 59 were flipped over toward the right in the drawing, the right electrode 54 then became an anode electrode, and the left electrode 56 became a cathode electrode.

Hydrogen peroxide ($H_2O_2$) and oxygen ($O_2$) were then produced from the hydroxide ions (OH-) in the right chamber 53. In addition, oxygen ($O_2$) was produced by the electrolysis of water, and chlorine gas ($Cl_2$) was produced from chlorine ions (Cl-). The preceding reaction can be expressed as follows.

$$2OH^- \rightarrow H_2O_2 + 2e^-$$

$$2OH^- \rightarrow H_2O + \frac{1}{2}O_2 + 2e^-$$

$$H_2O \rightarrow \frac{1}{2}O_2 + 2H^+ + 2e^-$$

$$2Cl^- \rightarrow Cl_2 + 2e^-$$

Hydroxide ions (OH-) and hydroxide-ion (OH-)-based compounds such as hydrogen peroxide ($H_2O_2$) were alternately produced by flipping the switches 58, 59 toward the right and left in the drawing.

Electrolyzed water containing hydroxide ions (OH-) and hydrogen peroxide ($H_2O_2$) was produced in the right chamber 53 after a fixed amount of time had elapsed. The reaction shown below presumably occurred in the electrolyzed water.

$$OH^- \rightarrow e^- + \cdot OH$$

$$H_2O \rightarrow H^+ + e^- + \cdot OH$$

$$H_2O_2 + H^+ + e^- \rightarrow H_2O + \cdot OH$$

(■OH) is a hydroxyl radical, and electrolyzed water containing hydroxyl radicals (■OH) was produced in the right chamber 53.

Although the measurement data has not been provided, the resulting electrolyzed water containing the hydroxyl radicals (■OH) retained its radical-related attribute even 28.5 hours after being produced. Accordingly, the electrolyzed water has a life of 24 hours or more, and is readily handled.

There will now be described an experimental example in which the aforedescribed electrolyzed water containing the hydroxyl radicals (■OH) was introduced, together with the cellulose, into the cellulose-lysing apparatus 30 shown in FIG. 3.

Experimental Examples

An experimental example according to the present invention will be described below; however, the present invention is not limited to the experimental example.

• Mixing step

Cellulose: microcrystalline, 2 g
Electrolyzed water: electrolyzed water containing hydroxyl radicals (■OH), 98 g

• Experiment to identify ideal pressure

The temperature was fixed at 200°C and the pressure was held at saturation vapor pressure or 5.0 MPa, and the resulting HMF (hydroxymethylfurfural) was examined. The results are shown in Table 3.

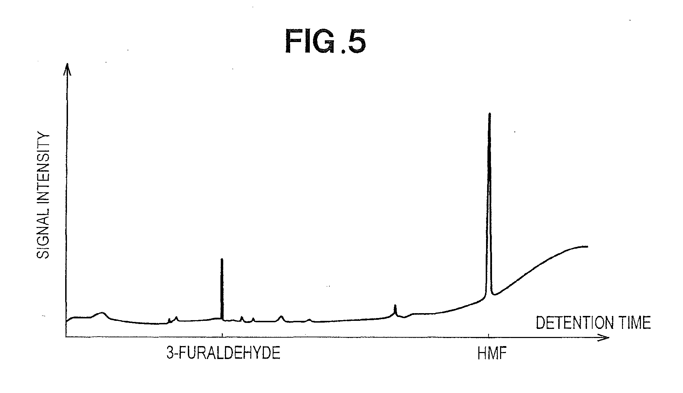

As shown in FIG. 5, the intensity of the signal for each component was detected when the stirred solution was subjected to TIC chromatography. The intensity of an HMF signal could be determined from among these.

Table 3

| Experiment | Mixture | | Processing | | | HMF |
|---|---|---|---|---|---|---|
| | Cellulose | Water | Temperature | Pressure | Time | |
| Experiment 11 | 2 g | Electrolyzed water containing hydroxyl radicals 98 g | 200°C | Saturation vapor pressure (1.6 MPa) | 30 min | Signal intensity 24.2 |
| Experiment 12 | 2g | Electrolyzed water containing hydroxyl radicals 98 g | 200°C | 5.0 MPa | 30 min | Signal intensity 15.5 |

In Experiment 11, electrolyzed water containing hydroxyl radicals (■OH) according to the present invention was added to cellulose, and the mixture was stirred for 30 minutes at 200°C and at saturation vapor pressure (1.6 MPa), whereupon HMF having a signal intensity of 24.2 was detected.

In Experiment 12, electrolyzed water containing hydroxyl radicals (■OH) according to the present invention was added to cellulose, and the mixture was stirred for 30 minutes at 200°C and at 5.0 MPa, whereupon HMF having a signal intensity of 15.5 was detected.

Although Experiment 12 (200°C, 5.0 MPa) was conducted at a high pressure, the signal intensity was less than that in Experiment 11 (200°C, saturation vapor pressure).

Accordingly, the recommended pressure is saturation vapor pressure.

The ideal temperature was then investigated at saturation vapor pressure and using electrolyzed water containing hydroxyl radicals (■OH).

• Experiment to identify ideal temperature

The pressure was held at saturation vapor pressure while the temperature was varied between 230 and 250°C, and the intensity of the HMF signal was examined. The results are shown in Table 4 below.

Table 4

| Experiment number | Mixture | | Processing conditions | | | HMF |
|---|---|---|---|---|---|---|
| | Cellulose | Water | Temperature | Pressure | Time | |
| Experiment 11 | 2 g | Electrolyzed water containing hydroxyl radicals 98 g | 200°C | Saturation vapor pressure (1.6 MPa) | 30 min | Signal intensity 24.2 |
| Experiment 13 | 2 g | Electrolyzed water containing hydroxyl radicals 98 g | 230°C | Saturation vapor pressure (2.8 MPa) | 30 min | Signal intensity 241.0 |
| Experiment 14 | 2 g | Electrolyzed water containing hydroxyl radicals 98 g | 250°C | Saturation vapor pressure (4.0 MPa) | 30 min | Signal intensity undetected |

Experiment 11 is a reproduction of Experiment 11 in Table 3.

In Experiment 13, electrolyzed water containing hydroxyl radicals (■OH) according to the present invention was added to cellulose, and the mixture was stirred for 30 minutes at 230°C and at saturation vapor pressure (2.8 MPa), whereupon HMF having a signal intensity of 241.0 was detected.

In Experiment 14, electrolyzed water containing hydroxyl radicals (■OH) according to the present invention was added to cellulose, and the mixture was stirred for 30 minutes at 250°C and at saturation vapor pressure (4.0 MPa), whereupon the signal intensity was imperceptible and no HMF was detected.

In Experiment 14, no increase was noted in the intensity of the HMF signal despite the temperature (250°C) being higher than that in Experiment 13 (230°C).

Accordingly, 230°C was identified as the ideal temperature at saturation vapor pressure.

As described in the prior art section, cellulose, a substance not readily lysed, has conventionally been processed at high temperatures (at or above 320°C) and high pressure (at or above 20 MPa).

However, according to the present invention, in which electrolyzed water containing hydroxyl radicals (■OH) is used, cellulose can be processed at a maximum temperature of 230°C and at a maximum pressure of 2.8 MPa (which corresponds to saturation vapor pressure).

Energy can be conserved because the temperature can be dramatically reduced (320°C to 230°C), and the cost of procuring processing equipment can be dramatically reduced because the pressure can be dramatically reduced (20 MPa to 2.8 MPa).

A method for obtaining a saccharide by lysing cellulose which is substance that is not readily lysed. Cellulose is mixed in acidic electrolyzed water, and the resulting mixture is stirred at a maximum temperature of 210°C and at saturation vapor pressure of 1.9 MPa to obtain a saccharide.

**Claims**

1.  A method for lysing cellulose, comprising the steps of:

    mixing the cellulose in acidic electrolyzed water; and
    stirring the resulting mixture at a maximum temperature of 210°C and at a saturation vapor pressure to obtain a saccharide.

2.  A method for lysing cellulose, comprising the steps of:

    mixing the cellulose in acidic electrolyzed water; and
    stirring the resulting mixture at a maximum temperature of 200°C and a maximum pressure of 2.0 MPa to obtain a saccharide.

3.  A method for lysing cellulose, comprising the steps of:

    mixing the cellulose in electrolyzed water containing a hydroxyl radical; and
    stirring the resulting mixture at a maximum temperature of 230°C and at a saturation vapor pressure to obtain hydroxymethylfurfural.

FIG.1

# FIG.2

# FIG.3

# FIG.4

# FIG.5

**EP 2 332 928 A2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2001095594 A